# FASCICULE DE BREVET EUROPEEN

(11) **EP 0 870 759 B1**
(45) Date de publication et mention de la délivrance du brevet: **21.03.2001**
(21) Numéro de dépôt: 98400811.0
(22) Date de dépôt: 06.04.1998
(51) Int. Cl.: C07C 225/20, A61K 31/13, C07C 233/78, C07C 235/50, A61K 31/16

(54) **Nouveaux composés aminés du 6,7,8,9-tétrahydro-cyclopenta a naphtalène et du 2,3-dihydro-cylopenta e indène, leur procédé de préparation et les compositions pharmaceutiques qui les contiennent**
Aminierte 6,7,8,9-Tetrahydro-cyclopenta[a]naphthalen- und 2,3-dihydro-cyclopenta[e]inden-Derivate, Verfahren zu ihrer Herstellung und diese enthaltende pharmazeutische Zubereitungen
Aminated 6,7,8,9-tetrahydro-cyclopenta[a]naphtalene- and 2,3-dihydro-cyclopenta[e]indene-derivatives, process for their preparation and pharmaceutical compositions containing them

(30) Priorité: 10.04.1997 FR 9704421
(43) Date de publication de la demande: 14.10.1998
(73) Titulaire: ADIR ET COMPAGNIE, 92415 Courbevoie Cédex (FR)
(72) Inventeur: Peglion, Jean-Louis, 78110 Le Vesinet (FR); Millan, Mark, 78230 Le Pecq (FR)

(56) Documents cités:
- CHEMICAL ABSTRACTS, vol. 91, no. 3, 16 juillet 1979 Columbus, Ohio, US; abstract no. 20185, HIRAOKA, MASAYUKI ET AL: "Cyclic aminoalcohols" XP002047874 & JP 54 003 047 A (FUNAI PHARMACEUTICAL INDUSTRIES, LTD., JAPAN)

## Description

La présente invention a pour objet de nouveaux composés aminés du 6,7,8,9-tétrahydro-cyclopenta[a]naphtalène et du 2,3-dihydro-cyclopenta[e]indène, leur procédé de préparation et les compositions pharmaceutiques qui les contiennent.

Elle concerne plus spécialement les composés de formule I : 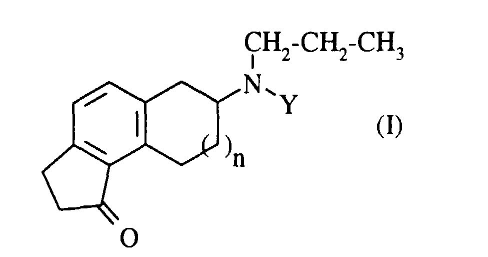 dans laquelle :
- n représente zéro ou un ; et
- Y représente un atome d'hydrogène ou un radical choisi parmi ceux répondant aux formules :

   -CH₂W,

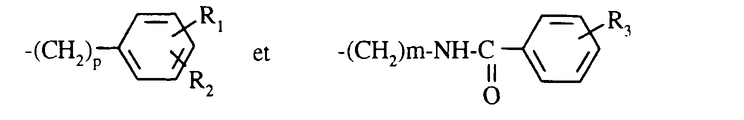

   dans lesquelles :
- W représente un atome d'hydrogène ou un radical alkyle, alkényle ou alkynyle ayant chacun de 1 à 5 atomes de carbone en chaine droite ou ramifiée,
- p représente un nombre entier de 1 à inclus,
- R₁ et R₂, identiques ou différents, représentent chacun un atome d'hydrogène ou d'halogène ou un radical hydroxy, (C₁-C₅) alkoxy, -NH-SO₂-CH₃, -NH-SO₂-CF₃, -NH-CO-CH₃, -NH-CO-CF₃ ou 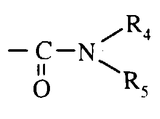 dans laquelle R₄ et R₅, identiques ou différents, représentent chacun un atome d'hydrogène ou un radical (C₁-C₆)alkyle en chaine droite ou ramifiée ;
- m représente un nombre entier de 2 à 5 inclus et
- R₃ représente un atome d'hydrogène ou d'halogène ou un radical hydroxy, (C₁-C₆) alkoxy, trifluorométhyle, cyano ou phényle ;
sous forme racémique ou d'isomères optiques et leurs sels d'addition avec un acide pharmaceutiquement acceptable.

Les composés de formule I agissent comme de puissants ligands dopaminergiques tant in vitro qu'in vivo. Parmi les cinq sous-types D₁ à D₅ de récepteurs dopaminergiques connus, les composés de la présente invention sont plus spécifiques vis à vis du sous-type réceptoriel dopaminergique D₃.

Actuellement, les substances utilisées en thérapeutique pour le traitement des affections dans lesquelles est impliqué le système dopaminergique, se lient toutes très fortement au sous-type réceptoriel D₂, qu'il s'agisse de bloqueurs dopaminergiques (utilisés dans les maladies imputables à l'hyperactivité de ce neurotransmetteur, comme par exemple dans la schizophrénie et les troubles psychotiques) ou d'activateurs dopaminergiques (utilisés dans les maladies imputables à l'hypoactivité de ce neurotransmetteur, comme par exemple dans la maladie de Parkinson). Toutefois ces bloqueurs ou activateurs dopaminergiques D₂ présentent de nombreux effets secondaires dyskinésie tardive, hyperprolactinémie, aménorrhée pour les premiers, effets cardio-vasculaires et moteurs pour les seconds.

Les récepteurs D₃, dont les concentrations sont très importantes au niveau du système limbique, mais contrairement aux récepteurs D₂ très faibles dans le noyau nigrostrié et dans les cellules lactotrophes, constituent donc un site d'action priviligié pour des molécules actives au niveau du système dopaminergique. Les molécules agissant préférentiellement au niveau des récepteurs dopaminergiques D₃, comme c'est le cas des composés de la présente invention, sont donc exemptes des effets secondaires liés typiquement aux ligands des récepteurs D₂, comme mentionnés précédemment.

En effet, des études réalisées in vitro (liaison sur récepteurs dopaminergiques clonés humains) avec les composés de la présente invention montrent que ceux-ci se comportent comme des ligands très affins au niveau des récepteurs dopaminergiques D₃.

Cette spécificité d'action confère aux composés de la présente invention un intérêt tout particulier pour leur utilisation comme médicament agissant au niveau du système dopaminergique, notamment dans la maladie de Parkinson (*J. Neur. Transm.,* **1993**, 94, 11-19), les troubles de la mémoire *(Nature,* **1990**, 347, 146-151), l'abus de drogue *(Science,* **1993**, 260, 1814), la dépression, la schizophrénie et les psychoses.

La présente invention a également pour objet les compositions pharmaceutiques contenant comme principe actif un composé de formule I ou un de ses sels physiologiquement tolérable mélangé ou associé à un ou plusieurs excipients pharmaceutiques appropriés.

Les compositions pharmaceutiques ainsi obtenues se présentent généralement sous forme dosée renfermant de 0,5 à 25 mg de principe actif. Elles peuvent, par exemple, revêtir la forme de comprimés, dragées, gélules, suppositoires, solutions injectables ou buvables et être administrées par la voie orale, rectale ou parentérale selon les formes utilisées.

La posologie varie selon l'âge et le poids du patient, la voie d'administration et les traitements associés et s'échelonne de 0,5 à 25 mg de principe actif, une à trois fois par jour.

La présente invention a également pour objet le procédé de préparation des composés racémiques ou optiquement actifs de formule I caractérisé en ce que l'on fait réagir
- une amine tertiaire de formule II : 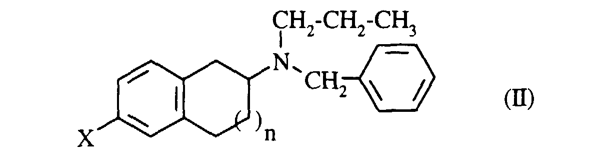 dans laquelle X est un atome d'halogène choisi parmi les atomes de brome et d'iode et n a la signification précédemment définie
   avec du butyllithium et de la diméthylformamide dans le tétrahydrofurane pour obtenir un aldhéhyde de formule III : 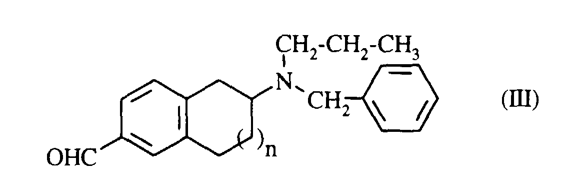 dans laquelle n a la signification précédemment définie,
   que l'on traite par l'acide malonique dans la pyridine en présence de pipéridine, pour conduire à un composé de formule IV : 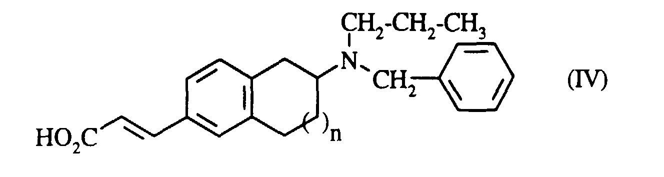 dans laquelle n a la signification précédemment définie,
   que l'on hydrogène en présence de palladium sur charbon, pour donner un acide de formule V : 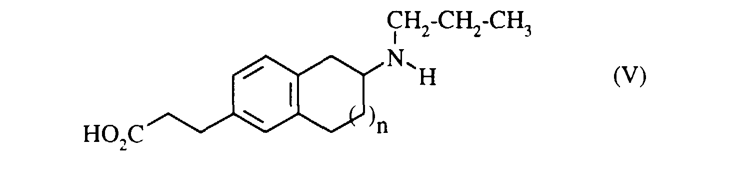 dans laquelle n a la signification précédemment définie,
   lequel est cyclisé au moyen d'acide polyphosphorique à chaud pour conduire à un composé de formule I': 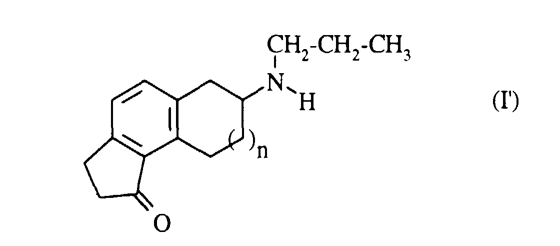 dans laquelle n a la signification précédemment définie,
   que l'on traite par un composé de formule VI :

   Hal-Y₁ (VI)

   dans laquelle Hal est un atome d'halogène choisi parmi les atomes de chlore, de brome et d'iode et Y₁ prend la signification de Y à l'exception de la valeur hydrogène pour conduire à un composé de formule I": 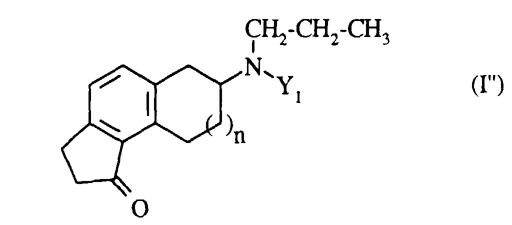 dans laquelle n a la signification précédemment définie et Y₁ a la signification de Y à l'exception de la valeur hydrogène.

L'ensemble des composés I' et II" constitue l'ensemble des composés de formule I.

Si on le désire, à partir des mélanges racémiques, on prépare les isomères optiques selon les méthodes classiques de la littérature.

Les sels des composés de formule I avec des acides pharmaceutiquement acceptables ont été obtenus selon des méthodes classiques comme indiquées dans les exemples ci-après. Les matières premières sont soit des produits connus, soit des produits obtenus à partir de substances connues, selon des méthodes connues de la littérature

Les exemples suivants, donnés à titre non limitatif, illustrent la présente invention. Les points de fusion ont été déterminés à la platine chauffante de Kofler sous microscope.

### EXEMPLE 1 : (7-R,S) 7-(N-propylamino)-6,7,8,9-tétrahydro-cyclopenta [a] naphthalén-1-one et son chlorhydrate

### Stade 1 : (7 R.S) 3-formyl-7-(N-benzyl-N-propylamino)-5,6,7,8-tétrahydronaphtalène

19,5g (0,0544 mole) de (7 R,S) 3-bromo-7-(N-benzyl N-propylamino)-5,6,7,8-tétrahydronaphthalène sont mis en solution dans 0,2431 de tétrahydrofurane et refroidi à -65° C. On coule, à cette température, 51 ml d'une solution 1,6 M de butyllithium dans le tétrahydrofurane. On laisse 2 heures après la fin de la coulée puis on additionne toujours à cette température une solution de 12,3 ml de diméthylformamide dans 28 ml de tétrahydrofurane. Après une heure à -65° C on abandonne à température ambiante pour un week-end. On reprend à l'eau et à l'éther, décante, sèche, évapore pour obtenir 16,3 g d'une huile qui correspond au produit attendu. (Rendement = 93 % )

### Stade 2 : acide 3-[(7 R,S) 7-(N-benzyl-N-propylamino)-5,6,7,8-tétrahydronaphthalén-3-yl] propénoïque

6,9g (0,022 mole) du composé obtenu au stade précédent, 11,3g (0,045 mole) d'acide malonique, 2,22 ml (0,022 mole) de pipéridine et 82,7 ml de pyridine sont amenés trois heures à 90° C, puis 2 heures à reflux. On abandonne la nuit à température ambiante et on concentre. On reprend par de l'acide chlorhydrique N et du chlorure de méthylène, on décante, sèche sur MgSO₄ et évapore pour obtenir 8g de cristaux fondant à 196° C et correspondant au produit attendu.

### Stade 3 : acide 3-[(7 R,S) 7-(N- propylamino)-5,6,7,8-tétrahydronaphthalén-3-yl] propionique

0,8g du composé obtenu au stade précédent, lg de formiate d'ammonium, 0,3g de Pd/C à 10% et 25 ml de méthanol sont portés au reflux durant 4 heures. Après filtration, on concentre le milieu réactionnel pour obtenir 0,4g d'un solide rose qui fond à 155°C et correspond au produit attendu. (Rendement = 74 %)

### Stade 4 : produit titre

3g du composé obtenu au stade précédent sont ajoutés en une seule fois dans 30 ml d'acide polyphosphorique chauffé préalablement à 75° C. La température s'élève alors jusqu'à 92° C. On maintient une heure à cette température. On coupe le chauffage et laisse redescendre la température à 45° C puis verse sur de la glace. On basifie avec de la soude concentrée et extrait à l'acétate d'éthyle. Après séchage et évaporation on obtient 1,4g d'une huile que l'on purifie par H.P.L.C. à l'aide d'une colonne nucléoprep 100-20, la phase mobile étant constituée d'un mélange de CH₂Cl₂/C₂H₅OH/ CF₃CO₂H dans le rapport 100/5/1. On obtient 0,73g de produit attendu (Rendement = 26,6 %) que l'on transforme en chlorhydrate par de l'éther chlorhydrique. P.F. du chlorhydrate attendu : 260-264 °C.

### EXEMPLE 2 : (7 R,S) 7-(N,N-dipropylamino)-6,7,8,9-tétrahydro-cyclopenta [a] naphthalén-1-one et son chlorhydrate

141mg (0,58 mmole) du composé titre de l'exemple 1 sont dissout dans 8,4 ml d'acétonitrile. On additionne ensuite 0,41g (3 mmole) de K₂CO₃ anhydre et 0,3 ml (3 mmole) d'iodure de propyle. On laisse sous agitation à température ambiante pendant 48 heures. On évapore le solvant puis reprend par de la soude 1N et de l'acétate d'éthyle. La phase organique est lavée à neutralité et séchée sur MgSO₄ puis évaporée pour conduire au produit attendu, qui est traité par de l'éther chlorhydrique pour donner 40mg de chlorhydrate. P.F. >260°C (Rendement = 21,5 %).

### EXEMPLE 3 : (7 R,S) 7-{N-propyl-N-[4-(o-trifluorométhylbenzamido)butyl] amino}-6,7,8,9-tétrahydro-cyclopenta [a] naphthalén-1-one et son chlorhydrate

A une solution de 0,7g (2,9 mmole) du composé titre de l'exemple 1 dans 50 ml de méthylisobutylcétone on ajoute 2g (15 mmole) de K₂CO₃ anhydre et 1,11 g (3,5 mmole) de N-(4-bromobutyl) o-trifluorométhylbenzamide. On porte à 75°C pendant 12 heures, puis après refroidissement on évapore le solvant, reprend à l'eau et au chlorure de méthylène, décante, sèche sur MgSO₄, évapore pour obtenir une huile qui est traitée par l'éther chlorhydrique pour donner 0,68g de chlorhydrate de produit attendu.

En opérant de la même façon qu'à l'exemple 3 ont été préparés les composés objet des exemples suivants :

### EXEMPLE 4 : (7 R,S) 7-{N-propyl-N-[4-(o-fluorobenzamido) butyl amino}-6,7,8,9-tétrahydro-cyclopenta [a] naphthalén-1-one

### EXEMPLE 5 : (7 R,S) 7-{N-propyl-N-[3-(o-méthoxybenzamido) propyl] amino}-6,7,8,9-tétrahydro-cyclopenta [a] naphthalén-1-one

### EXEMPLE 6:(7 R,S) 7-{N-propyl-N-[3-(o-bromobenzamido) propyl] amino}-6,7,8,9-tétrahydro-cyclopenta [a] naphthalén-1-one

### EXEMPLE 7 : étude pharmacologique

La sélectivité pour les récepteurs D₃ vis à vis des récepteurs D₂ a été démontrée :
- In vitro :: par la technique de liaison aux récepteurs D₂ et D₃.
- In vivo :: par la capacité des produits de l'invention à moduler l'hypothermie induite chez le rat par l'agoniste dopaminergique D₃ :7-OH-DPAT. (cf. : M.J. Millan et al., *J. Pharmacol. Exp. Ther.,* **1995**,275, 885).

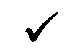 In vitro : étude de la liaison aux récepteurs humains D₂ et D₃

. Culture cellulaire
   Les cellules CHO (Chinese Hamster Ovary) ont été transfectées de façon stable par le gène codant pour le récepteur humain de la dopamine D₂ ou D₃ selon les méthodes connues de la littérature. Les cellules natives sont déficientes en enzyme DHFR (DiHydroFolate Reductase). Ces cellules sont cultivées dans une étuve à 37° C en atmosphère humide 5 % CO₂, 95 % air. Les transfections ont été réalisées en utilisant la Lipofectine (Gibco). Les cellules CHO cotransfectées avec le récepteur D₂ humain et le gène de résistance à la phléomycine ont été sélectionnées pour leur résistance à la présence de cet antibiotique dans le milieu de culture. Les cellules transfectées avec le récepteur D₃ humain ont été sélectionnées dans un milieu dépourvu d'hypoxanthine/thymidine, en présence de méthotréxate. La composition des milieux de culture utilisés sont pour les CHO-D₂ : DMEM (Dulbecco's Modified Eagle Medium) supplémentés à 10 % en sérum de veau foetal et en hypoxanthine/thimidine et pour les CHO-D₃ : DMEM supplémentés à 10 % en sérum de veau foetal dialysé. Les cellules sont récoltées à confluence et les membranes sont alors préparées.
. Préparation membranaire
   Après quelques minutes en présence de trypsine 0,2 %, les cellules sont récupérées et centrifugées à 2 000g pendant 5 minutes. Le culot cellulaire, resuspendu dans du tampon Tris-HCl 10 mM, pH 7,5 contenant 5mM de MgSO₄, est alors passé au Polytron®. L'homogénat est ensuite centrifugé à 50 000g pendant 15 minutes, et le culot est resuspendu par sonication douce dans le tampon d'incubation de composition : 50 mM de tris-HCl, pH 7,5 contenant 120 mM de NaCl, 5 mM de KCl, 2 mM de CaCl₂, 5 mM de MgCl₂. Les membranes sont ensuite aliquotées et conservées à -80° C jusqu'au jour de l'expérience.
. expériences de liaison
   Les incubations sont effectuées dans des tubes en polypropylène pour un volume final de 400 µl contenant :
   100 µl de [¹²⁵I]-iodosulpride (Amersham) à 0,1 et 0,2 nM pour les récepteurs D₂ et D₃ respectivement.
   100 µl de tampon (tubes totaux)
   ou 100 µl de raclopride 10 µM (liaison non spécifique)
   ou 100 µl de composé
   200 µl de préparation membranaire contenant les récepteurs D₂ ou D₃, dans du tampon additionné de 0,2 % de BSA (Bovine Serum Albumine).

   Les gammes de concentration de chaque composé comportent au moins sept points déterminés en triple, chaque expérience est répétée au moins deux fois.
   L'incubation qui dure trente minutes à 30° C est stoppée par une filtration rapide sur appareil Brandle, suivie de trois rinçages consécutifs avec du tampon Tris-HCl pH 7,4 contenant 120 mM de NaCl. Les filtres récupérés sont ensuite comptés au compteur gamma.
. Analyse des résultats
   L'IC₅₀ représentant la concentration donnant 50 % d'inhibition de la liaison du radioligand est calculée par régression non linéaire (méthode Prism Graph).
   La valeur de Kᵢ est dérivée de la formule Kᵢ = IC₅₀/(1+L/Kd) où L est la concentration de [¹²⁵I]-iodosulpride utilisée dans l'expérience et Kd sa constante de dissociation. Les résultats sont exprimés sous la forme de pKᵢ (pKᵢ = -logKᵢ).
   Pour les récepteurs humains D₂ et D₃, les Kd sont respectivement égaux à 0,5 et 1,31 nM.

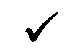 In vivo : hypothermie chez le rat

Les tests ont été réalisés sur des rats Wistar mâles de 200-250g placés en cage individuelle avec libre accès à la nourriture et à l'eau. Les produits ont été solubilisés dans de l'eau distillée, à laquelle plusieurs gouttes d'acide lactique sont ajoutées. Les injections ont été effectuées dans un volume de 1,0 ml/kg par voie sous-cutanée. Les doses sont exprimées en terme de base. La température rectale des rats a été enregistrée en utilisant un thermistoprobe digital (Millan et al., *J.P.E.T.,* **1993**, 264, 1364-1376). Dans un premier temps, les rats ont été injectés avec le composé à tester ou le véhicule, puis replacés dans leurs cages pendant trente minutes. Puis, les rats ont subi une injection de 7-OH-DPAT (0,16mg/kg) et ont été replacés dans leurs cages. Trente minutes plus tard, la température rectale a été mesurée et la différence a été déterminée par rapport aux valeurs basales (ΔT° C). La Dose Inhibitrice (95 % Limites de Confiance) pour réduire à 50 % l'effet du 7-OH-DPAT a été calculée selon la méthode de Finney (Statistical Method in Biological Assays, 2nd ed., Hafner publishing, New-York, 1964).

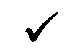 Résultats

Les produits de l'invention présentent une très bonne affinité pour le récepteur D₃. A titre d'exemple le composé de l'exemple 2 à un pKᵢ de 8,0.
De plus, les produits de l'invention montrent tous une sélectivité vis à vis du récepteur D₂ supérieure à un facteur 10.

## Revendications

1. Les composés aminés de formule I : 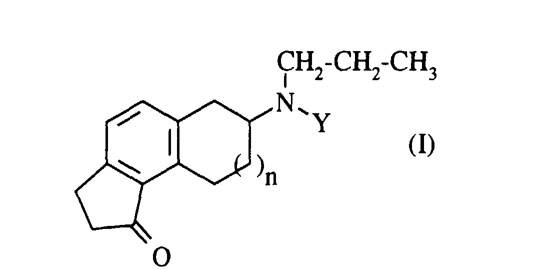 dans laquelle :
- n représente zéro ou un ; et
- Y représente un atome d'hydrogène ou un radical choisi parmi ceux répondant aux formules :
-CH₂W, 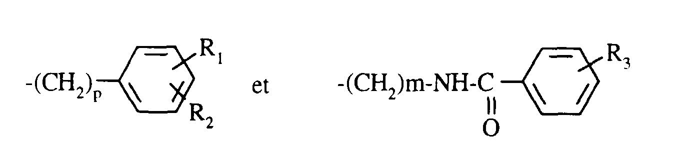
dans lesquelles :
- W représente un atome d'hydrogène ou un radical alkyle, alkényle ou alkynyle ayant chacun de 1 à 5 atomes de carbone en chaine droite ou ramifiée,
- p représente un nombre entier de 1 à 5 inclus,
- R₁ et R₂, identiques ou différents, représentent chacun un atome d'hydrogène ou d'halogène ou un radical hydroxy, (C₁-C₅) alkoxy, -NH-SO₂-CH₃, -NH-SO₂-CF₃, -NH-CO-CH₃, NH-CO-CF₃ ou 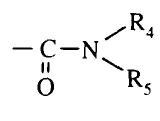 dans laquelle R₄ et R₅, identiques ou différents, représentent chacun un atome d'hydrogène ou un radical (C₁-C₆)alkyle en chaine droite ou ramifiée ;
- m réprésente un nombre entier de 2 à 5 inclus et
- R₃ représente un atome d'hydrogène ou d'halogène ou un radical hydroxy, (C₁-C₆) alkoxy, trifluorométhyle, cyano ou phényle ;
sous forme racémique ou d'isomères optiques et leurs sels d'addition avec un acide pharmaceutiquement acceptable.

2. Un composé de la revendication 1 choisi parmi le groupe constitué par les :
(7-R,S) 7-(N-propylamino)-6,7,8,9-tétrahydro-cyclopenta [a] naphthalén-1-one et son chlorhydrate,
(7 R,S) 7-(N,N-dipropylamino)-6,7,8,9-tétrahydro-cyclopenta [a] naphthalén-1-one et son chlorhydrate,
(7 R,S) 7-{N-propyl N-[4-(o-trifluorométhylbenzamido)butyl] amino}-6,7,8,9-tétrahydro-cyclopenta [a] naphthalén-1-one et son chlorhydrate,
(7 R,S) 7-{N-propyl-N-[4-(o-fluorobenzamido) butyl] amino}-6,7,8,9-tétrahydro-cyclopenta [a] naphthalén-1-one,
(7 R,S) 7-{N-propyl-N-[3-(o-méthoxybenzamido)propyl] amino}-6,7,8,9-tétrahydro-cyclopenta [a] naphthalén-1-one,
(7 R,S) 7-{N-propyl-N-[3-(o-bromobenzamido)propyl] amino}-6,7,8,9-tétrahydro-cyclopenta [a] naphthalén-1-one.

3. Un composé de la revendication 1 qui est la (7 R,S) 7-(N,N-dipropylamino)-6,7,8,9-tétrahydro-cyclopenta [a] naphthalén-1-one et son chlorhydrate.

4. Un composé de la revendication 1 qui est la (7 R,S) 7-{N-propyl-N-[4-(o-trifluorométhyl benzamido)butyl] amino} -6,7,8,9-tétrahydro-cyclopenta [a] naphthalén-1-one et son chlorhydrate.

5. Le procédé de préparation des composés racémiques ou optiquement actifs de la revendication 1 caractérisé en ce que l'on fait réagir :
- une amine tertiaire de formule II : 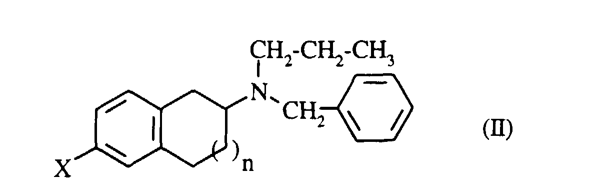 dans laquelle X est un atome d'halogène choisi parmi les atomes de brome et d'iode et n a la signification précédemment définie
avec du butyllithium et de la diméthylformamide dans le tétrahydrofurane pour obtenir un aldhéhyde de formule III : 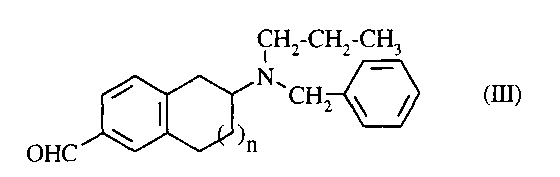 dans laquelle n a la signification précédemment définie,
que l'on traite par l'acide malonique dans la pyridine en présence de pipéridine, pour conduire à un composé de formule IV : 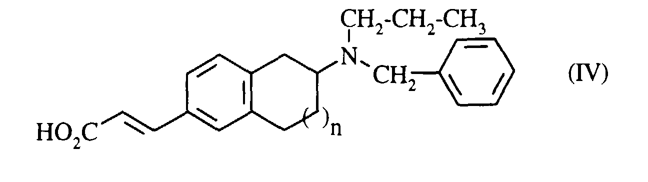 dans laquelle n a la signification précédemment définie,
que l'on hydrogène en présence de palladium sur charbon, pour donner un acide de formule V: 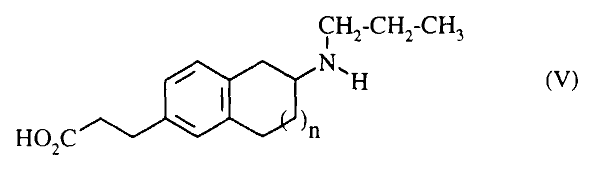 dans laquelle n a la signification précédemment définie,
lequel est cyclisé au moyen d'acide polyphosphorique à chaud pour conduire à un composé de formule I': 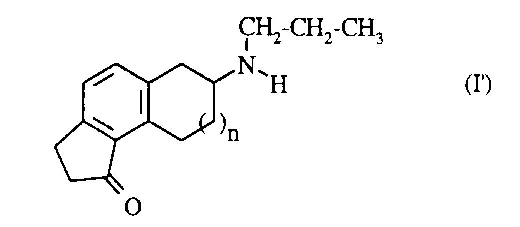 dans laquelle n a la signification précédemment définie,
que l'on traite par un composé de formule VI :
Hal-Y₁ (VI)
dans laquelle Hal est un atome d'halogène choisi parmi les atomes de chlore, de brome et d'iode et Y₁ prend la signification de Y à l'exception de la valeur hydrogène pour conduire à un composé de formule I": 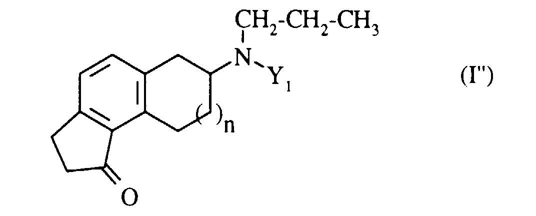 dans laquelle n a la signification précédemment définie et Y₁ a la signification de Y à l'exception de la valeur hydrogène,
et si on le désire, à partir des mélanges racémiques, on prépare les isomères optiques selon les méthodes classiques de la littérature,
et l'on transforme les composés de formule I en leurs sels d'addition avec des acides pharmaceutiquement acceptables.

6. Les compositions pharmaceutiques, utilisables dans le traitement de la maladie de Parkinson, des troubles de la mémoire, des troubles liés à l'abus de drogue, de la dépression, de la schizophrénie et des psychoses, contenant comme principe actif un composé selon une quelconque des revendications 1 à 4, avec un ou plusieurs excipients pharmaceutiques appropriés.

## Claims

1. The amino compounds of formula I: 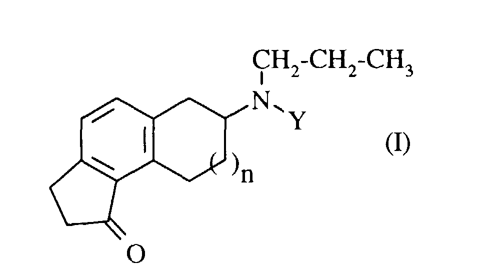 wherein :
- n represents zero or one ; and
- Y represents a hydrogen atom or a radical selected from those corresponding to formulae :
-CH₂W, 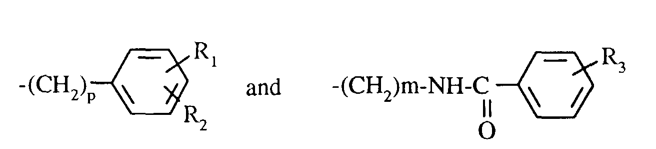
wherein :
- W represents a hydrogen atom or a straight-chain or branched alkyl, alkenyl or alkynyl radical each having from 1 to 5 carbon atoms,
- p represents an integer from 1 to 5 inclusive,
- R₁ and R₂, which may be the same or different, each represents a hydrogen or halogen atom or a hydroxy, (C₁-C₅)alkoxy, -NH-SO₂-CH₃, -NH-SO₂-CF₃, -NH-CO-CH₃, -NH-CO-CF₃ or 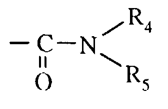 radical wherein R₄ and R₅, which may be the same or different, each represents a hydrogen atom or a straight-chain or branched (C₁-C₆)alkyl radical;
- m represents an integer from 2 to 5 inclusive, and
- R₃ represents a hydrogen or halogen atom or a hydroxy, (C₁-C₆)alkoxy, trifluoromethyl, cyano or phenyl radical ;
in racemic form or in the form of optical isomers, and addition salts thereof with a pharmaceutically acceptable acid.

2. A compound of claim 1 selected from the group consisting of:
(7R,S )-7-(N-propylamino)-6,7,8,9-tetrahydro-cyclopenta[a]naphthalen-1-one and the hydrochloride thereof,
(7R,S)-7-(N,N-dipropylamino)-6,7,8,9-tetrahydro-cyclopenta[a]naphthalen-1-one and the hydrochloride thereof,
(7R,S)-7-{N-propyl-N-[4-(o-trifluoromethylbenzamido)butyl]amino}-6,7,8,9-tetrahydro-cyclopenta[a]naphthalen-1-one and the hydrochloride thereof,
(7R,S)-7-{N-propyl-N-[4-(o-fluorobenzamido)butyl]amino}-6,7,8,9-tetrahydro-cyclopenta[a]naphthalen-1-one,
(7R,S)-7-{N-propyl-N-[3-(o-methoxybenzamido)propyl]amino}-6,7,8,9-tetrahydro-cyclopenta[a]naphthalen-1-one,
(7R,S)-7-{N-propyl-N-[3-(o-bromobenzamido)propyl]amino}-6,7,8,9-tetrahydro-cyclopenta[a]naphthalen-1-one.

3. A compound of claim 1 which is (7R,S)-7-(N,N-dipropylamino)-6,7,8,9-tetrahydro-cyclopenta[a]naphthalen-1-one and the hydrochloride thereof.

4. A compound of claim 1 which is (7R,S)-7-{N-propyl-N-[4-(o-trifluoromethylbenzamido)butyl]amino}-6,7,8,9-tetrahydro-cyclopenta[a]naphthalen-1-one and the hydrochloride thereof.

5. A process for the preparation of the racemic or optically active compounds of claim 1, characterised in that
- a tertiary amine of formula II : 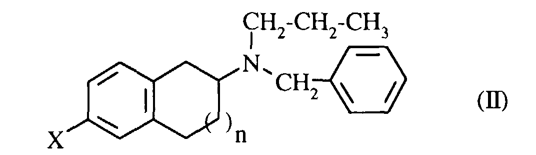 wherein X is a halogen atom selected from the atoms bromine and iodine and n is as defined above,
is reacted with butyllithium and dimethylformamide in tetrahydrofuran to obtain an aldehyde of formula III: 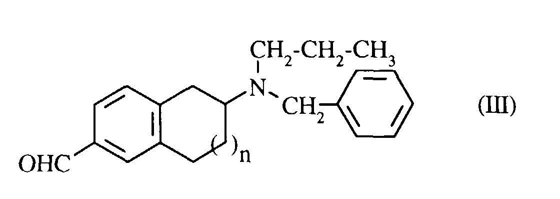 wherein n is as defined above,
which is treated with malonic acid in pyridine in the presence of piperidine to yield a compound of formula IV : 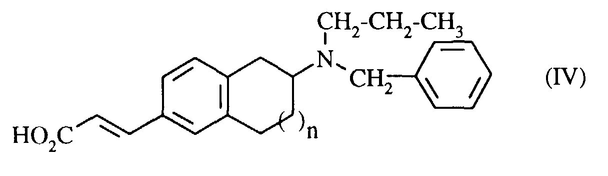 wherein n is as defined above,
which is hydrogenated in the presence of palladium-on-carbon to yield an acid of formula V: 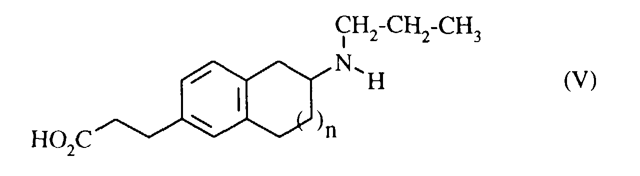 wherein n is as defined above,
which is cyclised using hot polyphosphoric acid to yield a compound of formula I': 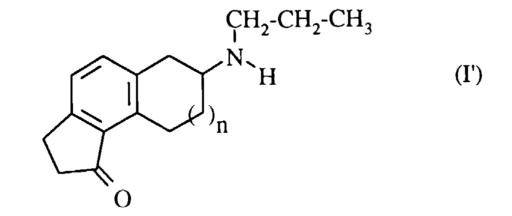 wherein n is as defined above,
which is treated with a compound of formula VI:
Hal-Y₁ (VI)
wherein Hal is a halogen atom selected from the atoms chlorine, bromine and iodine, and Y₁ has the meanings of Y, except for the meaning hydrogen, to yield a compound of formula I": 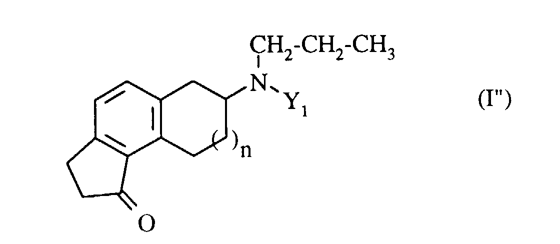 wherein n is as defined above and Y₁ has the meanings of Y except for the meaning hydrogen, and,
if desired, starting from the racemic mixtures, the optical isomers are prepared in accordance with conventional methods of the literature, and the compounds of formula I are converted into their addition salts with pharmaceutically acceptable acids.

6. Pharmaceutical compositions, for use in the treatment of Parkinson's disease, memory disorders, disorders associated with drug abuse, depression, schizophrenia and psychoses, comprising as active ingredient a compound according to any one of claims 1 to 4, with one or more suitable pharmaceutical excipients.

## Patentansprüche

1. Aminverbindungen der Formel I: 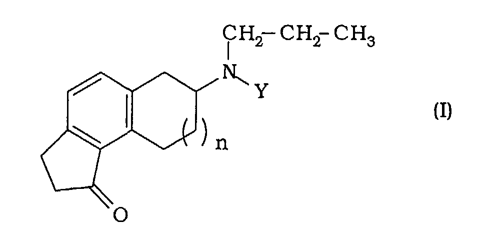 in der:
- n Null oder 1 bedeutet; und
- Y ein Wasserstoffatom oder eine Gruppe ausgewählt aus jenen, die den folgenden Formeln entsprechen, bedeutet:
-CH₂W, 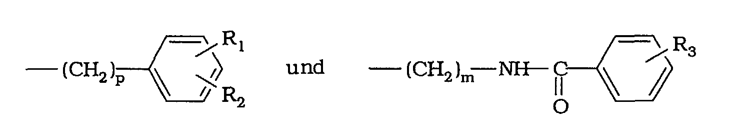
worin:
- W ein Wasserstoffatom oder eine Alkyl-, Alkenyl- oder Alkinylgruppe, die jeweils 1 bis 5 Kohlenstoffatome in gerader oder verzweigter Kette aufweist,
- p eine ganze Zahl mit einem Wert von 1 bis 5 einschließlich,
- R₁ und R₂, die gleichartig oder verschieden sind, jeweils ein Wasserstoffatom, ein Halogenatom, eine Hydroxygruppe, eine (C₁-C₅)-Alkoxygruppe, -NH-SO₂-CH₃, -NH-SO₂-CF₃, -NH-CO-CH₃, -NH-CO-CF₃ oder 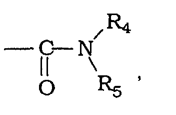 worin R₄ und R₅, die gleichartig oder verschieden sind, jeweils ein Wasserstoffatom oder eine verzweigte (C₁-C₆)-Alkylgruppe darstellen;
- m eine ganze Zahl mit einem Wert von 2 bis 5 einschließlich und
- R₃ ein Wasserstoffatom, ein Halogenatom, eine Hydroxygruppe, eine (C₁-C₆)-Alkoxygruppe, Trifluormethylgruppe, Cyanogruppe oder Phenylgruppe darstellt;
in racemischer Form oder in Form der optischen Isomeren sowie deren Additionssalze mit einer pharmazeutisch annehmbaren Säure.

2. Verbindung nach Anspruch 1, ausgewählt aus der Gruppe, die die folgenden Verbindungen umfaßt:
(7-R,S)-7-(N-Propylamino)-6,7,8,9-tetrahydro-cyclopenta[a]naphthalin-1-on und dessen Hydrochlorid,
(7 R,S)-7-(N,N-Dipropylamino)-6,7,8,9-tetrahydro-cyclopenta[a]naphthalin-1-on und dessen Hydrochlorid,
(7 R,S)-7-{N-Propyl-N-[4-(o-trifluormethylbenzamido)-butyl]-amino}-6,7,8,9-tetrahydro-cyclopenta[a]naphthalin-1-on und dessen Hydrochlorid,
(7 R,S)-7-{N-Propyl-N-[4-(o-fluorbenzamido)-butyl]-amino}-6,7,8,9-tetrahydro-cyclopenta[a]naphthalin-1-on,
(7 R,S)-7-{N-Propyl-N-[3-(o-methoxybenzamido)-propyl]-amino}-6,7,8,9-tetrahydro-cyclopenta[a]naphthalin-1-on,
(7 R,S)-7-{N-Propyl-N-[3-(o-brombenzamido)-propyl]-amino}-6,7,8,9-tetrahydro-cyclopenta[a]naphthalin-1-on.

3. Verbindung nach Anspruch 1, nämlich (7 R,S)-7-(N,N-Dipropylamino)-6,7,8,9-tetrahydro-cyclopenta[a]naphthalin-1-on und dessen Hydrochlorid.

4. Verbindung nach Anspruch 1, nämlich (7 R,S)-7-{N-Propyl-N-[4-(o-trifluormethylbenzamido)-butyl]-amino}-6,7,8,9-tetrahydro-cyclopenta[a]naphthalin-1-on und dessen Hydrochlorid.

5. Verfahren zur Herstellung der racemischen oder optisch aktiven Verbindungen nach Anspruch 1, dadurch gekennzeichnet, daß man:
- ein tertiäres Amin der Formel II: 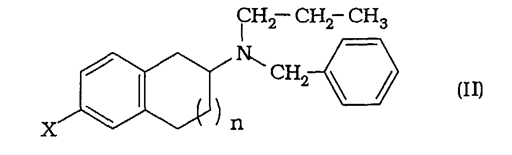 in der X ein Halogenatom ausgewählt aus Brom- und Iodatomen darstellt und n die oben definierten Bedeutungen besitzt,
mit Butyllithium und Dimethylformamid in Tetrahydrofuran umsetzt zur
Bildung eines Aldehyds der Formel III: 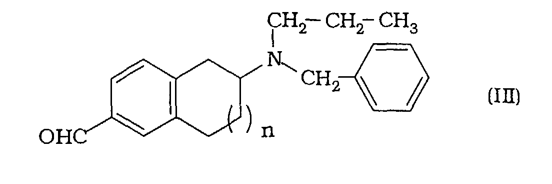 in der n die oben angegebene Bedeutung besitzt,
welchen man mit Malonsäure in Pyridin in Gegenwart von Piperidin behandelt zur Bildung einer Verbindung der Formel IV: 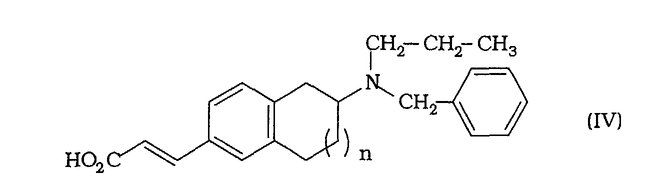 in der n die oben angegebene Bedeutung besitzt,
welche man in Gegenwart von Palladium-auf-Kohlenstoff hydriert zur Bildung einer Säure der Formel V: 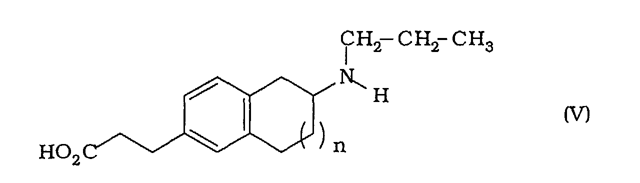 in der n die oben angegebene Bedeutung besitzt,
welche man mit Polyphosphorsäure in der Wärme cyclisiert zur Bildung einer Verbindung der Formel I': 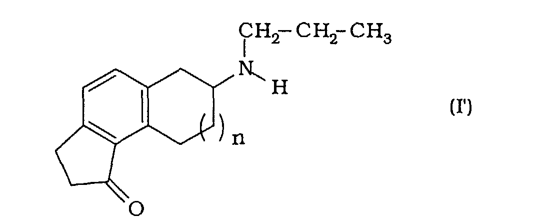 in der n die oben angegebene Bedeutung besitzt,
welche man mit einer Verbindung der Formel VI behandelt:
Hal - Y₁ (VI)
in der Hal ein Halogenatom ausgewählt aus Chlor-, Brom- und Iodatomen bedeutet und Y₁ die Bedeutungen von Y mit Ausnahme von Wasserstoff besitzt, zur Bildung einer Verbindung der Formel I": 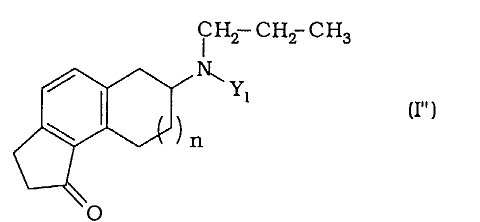 in der n die oben angegebene Bedeutung besitzt und Y₁ die Bedeutung von Y mit Ausnahme von Wasserstoff besitzt,
und man gewünschtenfalls ausgehend von den racemischen Mischungen die optischen Isomeren mit Hilfe klassischer Literaturverfahren bereitet, und die Verbindungen der Formel I mit pharmazeutisch annehmbaren Säuren in ihre Additionssalze überführt.

6. Pharmazeutische Zubereitungen für die Behandlung der Parkinsonschen Krankheit, von Gedächtnisstörungen, von Störungen, die mit dem Drogenmißbrauch verknüpft sind, von Depressionen, der Schizophrenie und Psychosen, enthaltend als Wirkstoff eine Verbindung nach einem der Ansprüche 1 bis 4 zusammen mit einem oder mehreren pharmazeutisch geeigneten Trägermaterialien.
